# EUROPEAN PATENT APPLICATION

(11) **EP 2 550 988 A1**
(43) Date of publication of application: **30.01.2013**
(21) Application number: 12177568.8
(22) Date of filing: 24.07.2012
(51) Int. Cl.: A61M 16/04

(54) **Loading dilator**

(30) Priority: 26.07.2011 US 201113190897
(71) Applicant: Cook Medical Technologies LLC, Bloomington, IN 47402-0489 (US)
(72) Inventor: Bosel, Christopher D., Bloomington, IN Indiana 47404 (US)
(74) Representative: Powell, Stephen David

(57) **Abstract**

A loading dilator (20) for positioning a medical apparatus (80) across an opening (94) formed through a body wall of a patient includes a tubular body having a relatively rigid proximal portion and a distal portion, the distal portion including a first relatively flexible segment (32) and a second relatively rigid segment (34). The first segment is axially extendable from a first length that has a greater diameter than the second segment, to a second length having a diameter that does not substantially exceed the diameter of the second segment. A stylet (50) is movable in a passageway of the tubular body between a first position wherein the first segment has the first length, and a second position wherein the stylet distal end exerts an axially displacing force on the second segment such that the first segment is movable from the first length to the second length.

## Description

### BACKGROUND

### Technical Field.

This application relates to a loading dilator for dilating an opening in the body of a patient for a medical use. More particularly, the invention relates to a loading dilator having a variable diameter transition segment at a distal portion thereof for use in positioning a medical device, such as a tracheostomy tube, across the body opening.

### 2. Background Information.

The creation of an adequate air passageway is a critical step in maintaining the ability of a seriously ill or injured patient to breathe, or in performing resuscitation on a patient unable to breathe. Endotracheal intubation (the placement of a tube through the nostrils or mouth and into the trachea itself) is a widely-used method for establishing an air passageway. However, in order to establish an optimal air passageway for endotracheal intubation, the trachea, nostrils and/or mouth must normally be free, or at least substantially free, of obstruction. When an obstruction is present, endotracheal intubation is not generally possible, and an alternative passageway for airflow must be established.

The most direct way to provide an air passageway under these circumstances is to form an opening, or stoma, through the tracheal wall. Once formed, a tracheostomy tube is inserted through the opening. Conventional tracheostomy tubes often include an open distal aperture and a circumferential inflatable cuff. The cuff provides a seal between the tracheal wall and the tracheostomy tube at a location proximal to the distal aperture. The seal prevents the intrusion of blood, tissue or foreign matter into the lower trachea, bronchi and lungs, while permitting complete control and monitoring of the airflow established through the tracheostomy tube, including the provision of positive pressure ventilation. The open distal aperture provides a passageway for air into the lungs of the patient.

Several methods and devices are known for forming, or enlarging, an opening in a tracheal wall. In one such method, a scalpel is used to form a small opening in the tracheal wall. A needle is inserted through the small opening, such that the tip of the needle is in the interior space of the trachea. A wire guide is then passed into the trachea through a bore in the needle, and the needle is thereafter withdrawn. Sequentially sized dilators are then advanced over the wire guide to facilitate gradual dilation of the tracheal entrance to an appropriate size.

Recently, a single curved dilator, sold by Cook Incorporated of Bloomington, Indiana, under the name BLUE RHINO®, has been developed that avoids the necessity of utilizing multiple dilators. The BLUE RHINO® dilator, so called because its shape resembles the horn of a rhinoceros, has a distal end portion that is curved in a substantially continuous manner, wherein an increasingly larger diameter portion of the dilator may be inserted into the trachea, thereby facilitating clearance of the posterior tracheal wall. Further description of the BLUE RHINO® dilator is provided in U.S. Patent No. 6,637,435, incorporated by reference herein.

Another method for forming or enlarging an opening in a tracheal wall for introduction of a tracheostomy tube is described in U.S. Patent No. 5,653,230, incorporated by reference herein. This method employs a balloon catheter having an inflatable balloon at a distal end of the catheter. The catheter is inserted over a percutaneously inserted wire guide, and the catheter is advanced along the wire guide until the balloon lies across the tracheal wall. The balloon is then inflated to radially dilate a portion of the tracheal wall, thereby forming an opening in the wall that corresponds to the inflated diameter of the balloon.

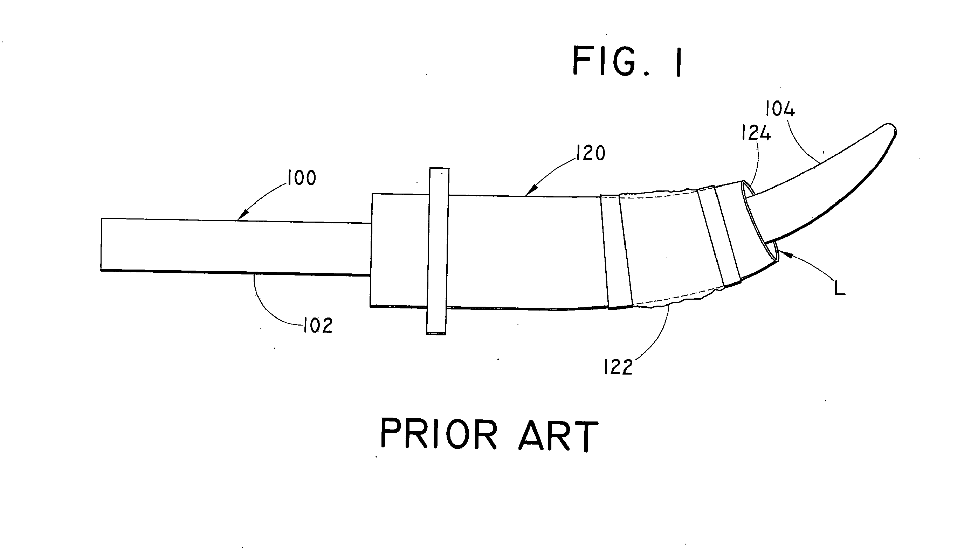

Following formation of the opening by any of the known methods, an introducer/loading dilator is pre-loaded with a tracheostomy tube, and the distal end of the apparatus is passed through the opening over the previously-inserted wire guide. It is desirable to provide a loading dilator/tracheostomy tube combination that has a generally smooth transition from loading dilator to tube, thereby facilitating entry of the distal, or leading, end portion of the tube through the opening. However, since there are a number of different sizes and manufacturers of tracheostomy tubes, there is a possibility that a significantly-sized lip (resulting from the respective differences in diameter between the loading dilator and the leading end of the tracheostomy tube) may be present at the transition between the loading dilator and the distal end of the tracheostomy tube. One example of a lip L is illustrated in Fig. 1 herein. The presence of a lip at a junction between a smaller diameter loading dilator and a larger diameter tracheostomy tube can hinder insertion of the tracheostomy tube through the opening, and can increase the trauma experienced by the patient upon insertion of the tube.

It would be desirable to provide a loading dilator that is sized to accommodate tracheostomy tubes having a range of diameters, and that is structured to minimize the transition between the loading dilator and the tracheostomy tube upon insertion of a dilator/tracheostomy tube apparatus.

### BRIEF SUMMARY

The problems of the prior art are addressed by the features of the present invention. In one form thereof, the invention comprises a loading dilator for positioning a tubular medical apparatus across an opening formed through a body wall of a patient, wherein the tubular medical apparatus is sized to fit over a portion of the loading dilator during positioning of the apparatus. The loading dilator comprises an elongated body having a proximal end, a distal end, a relatively rigid proximal portion extending in a distal direction from the proximal end, a distal portion extending in a proximal direction from the distal end, and a passageway between the proximal and distal ends. The distal portion has a first relatively flexible segment and a second relatively rigid segment. The second segment comprises at least one of a curved portion and a tapered portion. The first segment is positioned intermediate the proximal portion and the second segment along a length of the elongated body. The first segment is axially extendable from a first length wherein the first segment has a greater diameter than a diameter of the second segment, to a second length wherein the first segment has a diameter that does not substantially exceed the diameter of the second segment.

In another form thereof, the invention comprises an assembly for use in providing ventilation to a patient through an opening in the tracheal wall of a patient. The assembly comprises a loading dilator having an elongated body having a proximal end, a distal end, a relatively rigid proximal portion extending in a distal direction from the proximal end, a distal portion extending in a proximal direction from the distal end, and a passageway between the proximal and distal ends. The distal portion has a first relatively flexible segment and a second relatively rigid segment. The second segment has at least one of a curved portion and a tapered portion. The first segment is positioned intermediate the proximal portion and the second segment along a length of the elongated body and is selectively movable between a first length wherein the first segment has a greater diameter than a diameter of the second segment, and a second length wherein the first segment has a diameter that does not substantially exceed the diameter of the second segment. A stylet is received in the passageway. The stylet has a proximal end extending proximal of the elongated body proximal end, and a distal end. The stylet is movable in the passageway relative to the elongated body between a first position wherein the first segment has the first length, and a second position wherein the stylet distal end exerts an axially displacing force on the second segment such that the first segment is movable from the first length to the second length.

In yet another form thereof, the invention comprises a method for positioning a medical apparatus across an opening formed in a body wall of a patient. A loading dilator assembly is positioned to receive the medical apparatus thereon. The loading dilator assembly includes an elongated body having a proximal end, a distal end, a relatively rigid proximal portion extending in a distal direction from the proximal end, a distal portion extending in a proximal direction from said distal end, and a passageway between the proximal end and the distal end. The distal portion comprises a first relatively flexible segment and a second relatively rigid segment. The second segment has at least one of a curved portion and a tapered end portion. The first segment is positioned intermediate the proximal portion and the second segment along a length of the elongated body and is selectively movable between a first length wherein the first segment has a greater diameter than a diameter of the second segment, and a second length wherein the first segment has a diameter that does not substantially exceed the diameter of the second segment. A stylet is disposed in the passageway of the elongated body. The stylet has a proximal end extending proximal of the elongated body proximal end, and a distal end extending along the passageway. The stylet is advanced in a distal direction in the passageway from a first position wherein the first segment has the first length, to a second position wherein the stylet distal end exerts an axially displacing force relative to the second segment such that the first segment moves to the second length. The medical apparatus is advanced over an outer surface of the elongated body such that a leading end of the medical apparatus is positioned over the first segment. The stylet is withdrawn such that the axially displacing force is relaxed, whereby the first segment retreats from the second position and conforms to a position of the leading end of the medical apparatus, thereby forming a substantially nontaumatic transition between the elongated body distal end and the leading end of the medical apparatus.

### BRIEF DESCRIPTION OF THE DRAWINGS

Fig. 1 is a side view of a prior art loading dilator, wherein a tracheostomy tube is loaded onto the dilator;
Fig. 2 is a side view of a loading dilator and stylet, according to an embodiment of the present invention;
Fig. 2A is an enlarged view of a portion of loading dilator of Fig. 2;
Fig. 3 is a proximal end view of the loading dilator and stylet of Fig. 2;
Fig. 4 is a side view of the loading dilator and stylet as in Fig. 2, after a force has been applied to the proximal end of the stylet;
Fig. 5 is a side view of a loading dilator assembly according to an embodiment of the present invention, showing a tracheostomy tube loaded onto a surface of the loading dilator;
Fig. 6 is a side view of a loading dilator assembly as in Fig. 5, including a blunt-tipped tracheostomy tube; and
Fig. 7 is a view illustrating the use of a loading dilator assembly according to the present invention in positioning a tracheostomy tube across a tracheal wall.

### DETAILED DESCRIPTION OF THE DRAWINGS AND THE PRESENTLY PREFERRED EMBODIMENTS

For the purposes of promoting an understanding of the principles of the invention, reference will now be made to the embodiments illustrated in the drawings, and specific language will be used to describe the same. It should nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

In the following discussion, the terms "proximal" and "distal" are used to describe the axial ends of the loading dilator, as well as the axial ends of related components. The "proximal" end is used in conventional manner to refer to the end of the dilator (or component) that is closest to the operator during use of the loading dilator. The "distal" end is used in conventional manner to refer to the end of the loading dilator (or component) that is initially inserted into the patient, or that is closest to the patient.

Fig. 1 illustrates a side view of a prior art loading dilator 100. In the figure, a tracheostomy tube 120 having an inflatable cuff 122 (shown in the uninflated condition) and a distal end 124 is loaded onto the outer surface of the loading dilator for placement across the tracheal wall of a patient.

Prior art loading dilator 100 includes an elongated body 102 having a distal portion 104 that is tapered for ease of entry into the dilated opening previously formed in the tracheal wall. A passageway (not shown) extends through loading dilator 100 for passage of a wire guide (not shown) therethrough. Typically, elongated dilator body 102 is gently curved at the distal end portion to ease entry of the tracheostomy tube through the tracheal wall, and to generally conform to the anatomy within the cavity of the trachea.

In order to accommodate patients of varying sizes, loading dilators and tracheostomy tubes are provided in a variety of different diameters. Ideally, the respective diameters of the loading dilator and the tracheostomy tube will be substantially matched, such that there is only a minimal diametrical transition between the loading dilator and the distal end of the tracheostomy tube. As a result, the trauma experienced by the patient upon insertion of the tracheostomy tube through the tracheal wall will be minimized. However, due to the wide variance in diameters between the loading dilators and tracheostomy tubes that may be available to the physician in the operating room at any one time, it may not be possible to closely match the diameters of the respective loading dilators and/or tracheostomy tubes available to the physician. In some cases, selection of an available loading dilator and tracheostomy tube may result in the presence of a lip, or a significant diametrical difference between the loading dilator and the distal end of the tracheostomy tube at the transition.

A loading dilator/tracheostomy tube combination having a lip "L" is shown in the prior art combination of Fig. 1. The lip L shown in Fig. 1 is somewhat exaggerated from that which may typically be expected, and is shown in the figure to aid in identifying the position of the transition referenced herein. The presence of a lip of any size at the transition of the dilator 100 and the distal end 124 of the tracheostomy tube 120 is likely to cause at least some difficulty of insertion and/or trauma to the patient upon introduction of the tracheostomy tube through the tracheal wall. The presence of a larger lip is likely to cause significant difficulty of insertion and/or patient trauma.

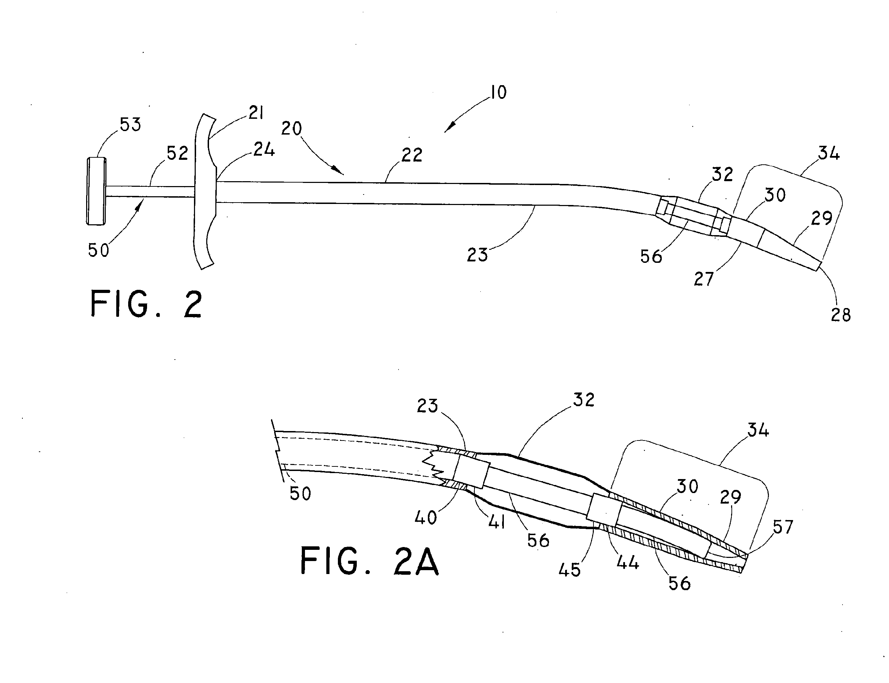

Fig. 2 is a side view of a loading dilator assembly 10 according to an embodiment of the present invention. Loading dilator assembly 10 includes a loading dilator 20, and an elongated member, such as stylet 50, slidably received in a passageway extending through the loading dilator.

In the embodiment shown, loading dilator 20 comprises an elongated body 22 having a proximal portion 23 and a distal portion 27. Proximal portion 23 extends to a proximal end 24, and distal portion 27 extends to a distal end 28. Distal portion 27 comprises a first segment 32 and a second segment 34, as further described herein. A finger grip element, such as finger grip 21, is affixed to loading dilator proximal end 24.

Proximal portion 23 of the dilator body is formed from any relatively rigid, medical grade, synthetic material known in the art for such use in forming a loading dilator. PVC and polyurethane are two non-limiting examples of suitable materials. Proximal portion 23 may have a length of about 12 to 18 cm, although those skilled in the art will appreciate that this length may be varied if desired.

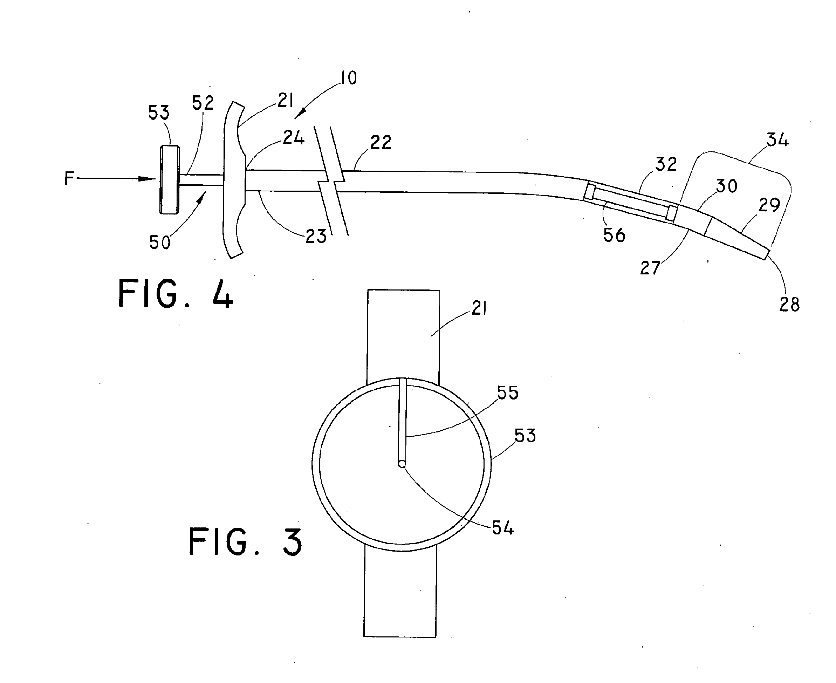

As stated above, distal portion 27 comprises first and second segments 32 and 34. As shown in Figs. 2, 2A, and 4, first segment 32 is positioned intermediate proximal portion 23 and second segment 34 along the length of elongated body 22. Second segment 34 is also formed from a relatively rigid, medical grade, synthetic material. Although not required, proximal portion 23 and second segment 34 are preferably formed of the same or a similar composition, and are preferably of substantially similar rigidity. Second segment 34 preferably has a generally curved or offset portion 30 (relative to proximal portion 23), and a tapered portion 29 that extends to distal end 28. Curved or offset portion 30 (hereafter "curved") and tapered portion 29 are well known features of loading dilators, and those skilled in the art are readily able to determine a suitable degree of curvature, offset, and taper for a particular loading dilator having either, or both, of these features.

First segment 32 is formed from a material, such as silicone, having a lower rigidity (i.e., a greater flexibility) than the material of proximal portion 23 and second segment 34. First segment 32 has sufficient elasticity to allow axial extension upon application of a force thereon. Other non-limiting examples of suitable materials for first segment 32 include low durometer polyurethanes as well as various thermoplastic elastomers having sufficient capability for stretching as described herein. Those skilled in the art will appreciate that other biocompatible elastic materials capable of flexure and extension as described may also be substituted for silicone.

When first segment 32 is in the relaxed configuration shown in Fig. 2, this segment has a larger outer diameter than the outer diameter of the adjoining curved portion 30, such as between about 2 to 5 mm, and typically about 3 mm, greater than the outer diameter of portion 30. Thus, for example, in one preferred embodiment, portion 30 has an outer diameter of about 7 mm and first segment 32 has an outer diameter of about 10 mm in the relaxed configuration shown in the figure. Preferably, proximal portion 23 and curved portion 30 will have the same or a substantially similar outer diameter. In this case, first segment 32 will have a larger diameter (by about 3 mm) than both proximal portion 23 and portion 30, as shown in Figs. 2 and 2A. Typically, first segment 32 is constructed to have a relaxed length of about 3-5 cm, preferably about 4 cm, and second segment 34 will have a total length of about 1 to 3 cm, preferably about 2 cm. Those skilled in the art will appreciate the dimensions provided hereinabove are only examples of suitable lengths, and other dimensions may be suitable for a particular case.

In order to interconnect the proximal portion 23, first segment 32, and second segment 34, as shown, the respective proximal and distal ends of flexible first segment 32 may be affixed to corresponding axial ends of proximal portion 23 and second segment 34. Those skilled in the art will appreciate that there are many suitable ways to accomplish these connections in a secure manner. In one such manner, small diameter plastic tubing, such as cannulae 40, 44 (Fig. 2A), are snugly received in the respective passageways (not shown) extending through proximal portion 23 and second segment 34, respectively. In one preferred embodiment, the plastic tubing or cannula is formed of the same or a similar material as dilator proximal portion 23 and/or distal second segment 34. Alternatively, cannulae 40, 44 may be formed of other generally rigid biologically compatible materials, such as polypropylene, nylon, or thermoplastics such as acrylonitrile butadiene styrene (ABS). Cannula 40 is affixed in proximal portion 23 such that cannula distal portion 41 extends outwardly (distally) of the proximal portion. Cannula 44 is affixed in second segment 34, such that cannula proximal portion 45 extends outwardly (proximally) of this segment. Cannulae 40, 44 may be securely affixed in the respective portion of the loading dilator by known means, such as by use of an adhesive, by mechanical bonding, or by suitable physical connection, e.g., a threaded connection.

A suitably-sized segment 32 of silicone or other flexible material having a passageway extending therethrough is fitted between proximal portion 23 and second segment 34. The respective ends of segment 32 securely receive cannulae ends 41, 45. The respective ends of this segment may then be affixed to the corresponding ends of proximal portion 23 and second segment 34, and to respective cannulae ends 41, 45 by known means, such as by an adhesive or mechanical bonding. Each of the outer axial ends of first segment 32 (approximately 0.5 cm of each end) is preferably tapered or chamfered prior to assembly to provide a smooth transition between the ends of segment 32 and respective proximal portion 23 and second distal segment 34 as shown in Figs. 2, 2A. By fitting the ends of segment 32 over respective cannulae ends 41, 45, additional surface area is provided to ensure a secure connection between the ends of first segment 32, and proximal portion 23 and second segment 34, respectively.

As stated above, an elongated generally rigid member, such as stylet 50, is slidably received in the passageway extending along the interior of elongated body 22. As shown in Figs. 2 and 4, proximal portion 52 of stylet 50 extends proximal of loading dilator proximal end 24. Stylet 50 may include a thumb or palm button 53 at the extreme proximal end of the stylet, as further described herein. As shown in Fig. 3, stylet 50 (including button 53) may have a passageway 54 extending therethrough for receiving a wire guide. Button 53 may also be provided with a slot 55 communicating with passageway 54 to permit the wire guide to be re-aligned during activation of the stylet. Stylet 50 is sized such that a distal portion 56 of the stylet extends through the passageway in loading dilator 20, whereby the extreme distal end 57 of the stylet abuts the interior wall of second segment 34, e.g., at tapered portion 29 (Fig. 2A). The stylet can be provided with a slight distal curve to facilitate engagement with the distal tip portion of the dilator.

Initially, the proximal end of the stylet (e.g., at button 53) extends a distance of about 4-5 cm proximal of loading dilator proximal end 24, as shown in Fig. 2. At this time, stylet distal end 57 touches, or is closely spaced from, the interior wall of the loading dilator elongated body at second segment 34, as described above and as shown in Fig. 2A. Utilizing the finger grip 21 as a counterforce, the operator positions her thumb/palm on stylet button 53, and exerts a distally-directed axial force F on button 53 (Fig. 4). At this time, stylet 50 is urged in the distal direction within the passageway of loading dilator elongated body 22, such that stylet distal end 57 exerts a pushing force against segment 34. As a consequence of this force, loading dilator second segment 34 is pushed in a distal direction, thereby causing first segment 32 to displace, or stretch, from the relaxed position shown in Figs. 2, 2A, to the lower diameter stretched position shown in Fig. 4.

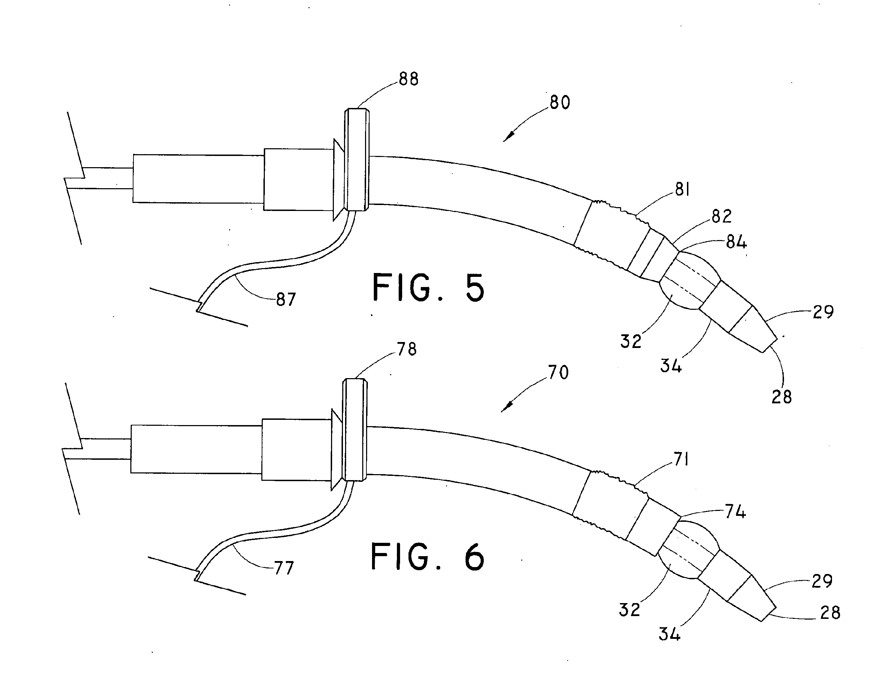

As stated, loading dilator assembly 10 may be used for inserting a tubular medical apparatus across an opening formed through a body wall of a patient. One example of such use is the insertion of a tracheostomy tube 80 across an opening formed through the tracheal wall. Tracheostomy tubes are well known in the medical arts, and tracheostomy tube 80 may be of any size and shape commonly utilized in the art. In the embodiment shown in Fig. 5, tracheostomy tube 80 has a tapered distal portion 82 that extends to a distal, or leading, end 84. Tracheostomy tube 80 has a generally curved body, and an inflatable cuff 81 (shown in the uninflated condition in Fig. 5). Cuff 81 may be inflated in conventional fashion, e.g., via passage of an inflation fluid through cuff inflation tube 87. When properly positioned in the trachea, the inflatable cuff provides a seal between the tracheal wall and the tracheostomy tube to prevent the intrusion of blood, tissue, and other foreign matter in the lower trachea, bronchi, and lungs. The open distal end 84 provides a passageway for air into the lungs of the patient. A conventional flange 88 may be provided at the proximal end of the tracheostomy tube for abutment against the skin of the patient when tracheostomy tube 80 is positioned in the tracheal opening.

When utilized in combination with loading dilator assembly 10, tracheostomy tube 80 (Fig. 5) is initially advanced over the loading dilator assembly when first segment 32 is in the extended, or stretched, position, as shown in Fig. 4. Lubricating jelly may be applied to the inside of the tracheostomy tube and/or the outside of the dilator in the same manner as applied to existing devices. The tracheostomy tube is advanced relative to the loading dilator assembly such that leading end 84 is positioned over stretched first segment 32, preferably about midway along the length of the segment. When stylet 50 is withdrawn in the proximal direction, the axially displacing force F exerted upon the curved portion of second segment 34 is released. Upon release of force F, segment 32 has a tendency to return to the relaxed position shown in Figs. 2, 2A. Due to the presence of the tracheostomy tube, and particularly leading end 84, full return of first segment 32 to the original diameter is prevented. At this time, flexible first segment 32 assumes a somewhat bulbous configuration relative to tracheostomy tube leading end 84, as shown in Fig. 5. This configuration eliminates the abrupt transition occasioned by the presence of lip L as shown in Fig. 1, and instead, provides a generally smooth transition for the leading end of the tracheostomy tube upon entrance through the opening in the tracheal wall. Thus, a smoother and less traumatic entrance of the tracheostomy tube into the opening in the tracheal wall is achieved.

The following example provides additional details of the use of the loading dilator assembly in combination with a tracheostomy tube. Those skilled in the art will appreciate that with minor modification to the process steps described, the loading dilator assembly can be utilized to insert other tubular medical apparatuses across openings formed in other body walls.

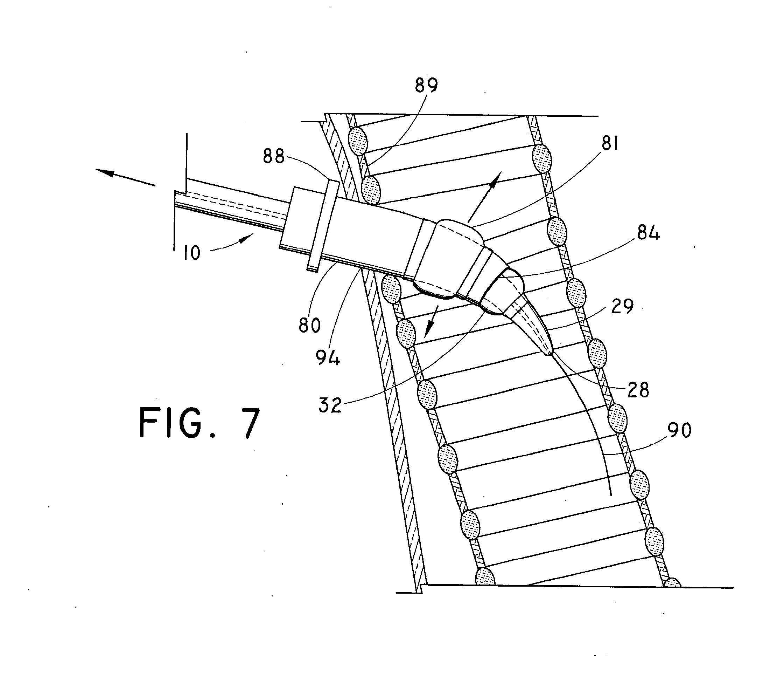

The insertion of a tracheostomy tube through an opening in a tracheal wall is a well-known technique, and as such, the skilled artisan is well equipped to determine appropriate means for forming a suitable opening through the tracheal wall. In one known technique, a wire guide 90 (Fig. 7) is percutaneously inserted through the tracheal wall, e.g., through the interior of a previously-inserted hollow needle (not shown). Following removal of the needle, the distal end of the wire guide remains in place across the tracheal wall 89. The opening 94 may then be dilated using, e.g., a dilator such as the curved BLUE RHINO® dilator described in the incorporated by reference U.S. Patent No. 6,637,435, or the balloon dilator as described in the incorporated by reference U.S. Patent No. 5,653,230.

A loading dilator assembly 10 as described hereinabove is arranged for insertion into the opening 94 formed through the tracheal wall. Stylet 50 is axially advanced with force F to axially displace loading dilator first segment 32 from the relaxed position as shown in Figs. 2, 2A to the extended position shown in Fig. 4. Tracheostomy tube 80 is then loaded onto the outer surface of loading dilator 10 as described above, and advanced relative to the loading dilator such that leading end 84 is positioned over first segment 32 as described above. Stylet 50 is withdrawn, such that segment 32 retreats to the bulbous-like configuration shown in Fig. 5.

At this time the assembly 10, including tracheostomy tube 80 fitted thereover, is positioned for advancement into tracheal opening 94. The proximal end of wire guide 90 is back loaded into loading dilator distal end 28, and threaded through the assembly. The assembly and tracheostomy tube are advanced over the wire guide in well-known fashion, until the tracheostomy tube is properly positioned along the tracheal wall as shown in Fig. 7. With first segment 32 in the configuration as shown, a substantially non-traumatic diametrical transition is created between loading dilator 20 and the leading distal end 84 of tracheostomy tube 80.

Once insertion is complete and the tracheostomy tube has been properly positioned, the loading dilator assembly may be removed. Axial force F is once again asserted on button 53, so that stylet 50 is once again urged in the distal direction to displace first segment 32 to the stretched position shown in Fig. 4. As a result, the outer diameter of segment 32 is reduced such that the loading dilator assembly 10 (loading dilator and stylet) can be withdrawn from the interior of the tracheostomy tube 80, leaving the tracheostomy tube in place across the tracheal wall. Tracheostomy tube cuff 81 may then be inflated, and the tracheostomy tube may be secured in place by well-known means.

As stated above, tracheostomy tubes are well known in the medical arts, and are provided in many different sizes and shapes. In the embodiment illustrated in Fig. 5 and described hereinabove, tracheostomy tube 80 has a tapered distal portion 82 that extends to leading end 84. Tubes having this tapered portion are typically used in percutaneous insertion as described above.

Another common tracheostomy tube configuration is shown in Fig. 6. Tracheostomy tube 70 is generally similar to that of tracheostomy tube 80, and includes a generally curved body, a cuff 71, and a flange 78. However, rather than providing the tapered distal end as shown in Fig. 5, tracheostomy tube 70 is provided with a blunt leading end 74. Tracheostomy tubes having a blunt tip are typically utilized in surgical insertion. Notwithstanding the presence of a blunt leading end on the tracheostomy tube 70 shown in Fig. 6, loading dilator first segment 32 may be stretched, and released, to form the generally tapered, bulbous-like leading end in the same manner as with tracheostomy tube 80 shown in Fig. 5. Thus, the loading dilator as described will find application in tracheostomy tubes inserted both percutaneously and surgically.

Those skilled in that art will appreciate that the foregoing detailed description should be regarded as illustrative rather than limiting, and that it should be understood that it is the following claims, including all equivalents, that are intended to define the scope of this invention.

The features of the various embodiments may be combined or interchanged as desired.

The disclosures in United States patent application no. 13/190,897, from which this application claims priority, and in the abstract accompanying this application are incorporated herein by reference.

## Claims

1. A loading dilator for positioning a tubular medical apparatus across an opening formed through a body wall of a patient, the tubular medical apparatus being sized to fit over a portion of the loading dilator during said positioning of the apparatus, the loading dilator comprising:
an elongated body having a proximal end, a distal end, a relatively rigid proximal portion extending in a distal direction from said proximal end, a distal portion extending in a proximal direction from said distal end, and a passageway between said proximal end and said distal end, said distal portion having a first relatively flexible segment and a second relatively rigid segment, said second segment comprising at least one of a curved portion and a tapered portion, said first segment intermediate said proximal portion and said second segment along a length of said elongated body, said first segment axially extendable from a first length wherein said first segment has a greater diameter than a diameter of the second segment to a second length wherein said first segment has a diameter that does not substantially exceed said diameter of the second segment.

2. The loading dilator of claim 1, wherein each of said proximal portion, first segment, and second segment have respective axial ends, said proximal portion and said segment securely affixed at adjoining axial ends thereof, and said first segment and said second segment securely affixed at adjoining axial ends thereof.

3. The loading dilator of claim 2, wherein a distal end portion of said first segment tapers toward said second segment when said first segment has said first length.

4. The loading dilator of claim 3, wherein a proximal end portion of said first segment tapers toward said proximal portion when said first segment has said first length.

5. The loading dilator of any preceding claim, further comprising a finger grip at said elongated body proximal end.

6. The loading dilator of any preceding claim, further comprising a stylet received in said passageway.

7. The loading dilator of any preceding claim, wherein said first segment has an outer diameter about 2 to 5 mm greater than said outer diameter of the second segment, when said first segment is at said first length.

8. The loading dilator of any preceding claim, wherein a proximal end of said first segment is affixed to a distal end of said proximal portion, and a distal end of said first segment is affixed to a proximal end of said second segment.

9. The loading dilator of claim 8, further comprising a first cannula positioned in said passageway at a juncture of said proximal portion and said first segment, and a second cannula positioned in said passageway at a juncture of said first segment and said second segment.

10. The loading dilator of any preceding claim, wherein said proximal portion and said second segment are formed of a substantially similar composition and have a substantially similar outer diameter, and wherein said first segment is formed of silicone.

11. An assembly for use in providing ventilation to a patient through an opening in the tracheal wall of a patient, comprising:
a loading dilator comprising an elongated body having a proximal end, a distal end, a relatively rigid proximal portion extending in a distal direction from said proximal end, a distal portion extending in a proximal direction from said distal end, and a passageway between said proximal end and said distal end, said distal portion having a first relatively flexible segment and a second relatively rigid segment, said second segment having at least one of a curved portion and a tapered portion, said first segment positioned intermediate said proximal portion and said second segment along a length of said elongated body and being selectively movable between a first length wherein said first segment has a greater diameter than a diameter of said second segment, and a second length wherein said first segment has a diameter that does not substantially exceed the diameter of the second segment; and
a stylet received in said passageway, said stylet having a proximal end extending proximal of said elongated proximal end, and a distal end, said stylet movable in said passageway relative to said elongated body between a first position wherein said first segment has said first length, and a second position wherein said stylet distal end exerts an axially displacing force on said second segment such that said first segment is movable from said first length to said second length.

12. The assembly of claim 11, further comprising a tracheostomy tube carried on an outer surface of the loading dilator, said tracheostomy tube sized to be received in said tracheal wall opening.

13. The assembly of claim 12, wherein said second segment tapers in a direction of the distal end of said loading dilator, and wherein said first segment defines a substantially nontaumatic transition between said elongated body second segment and a leading end of the tracheostomy tube.

14. The assembly of claim 12 or 13, wherein said tracheostomy tube has a tapered portion, and said stylet exerts said force on said tapered portion.

15. The assembly of any of claims 11 to 14, wherein said stylet includes a passageway therethrough, further comprising a wire guide extending through said stylet.
